(19) Europäisches Patentamt

European Patent Office

Office européen des brevets

(11) **EP 0 885 423 B1**

(12) **EUROPÄISCHE PATENTSCHRIFT**

(45) Veröffentlichungstag und Bekanntmachung des Hinweises auf die Patenterteilung:
**17.11.1999 Patentblatt 1999/46**

(21) Anmeldenummer: **97915304.6**

(22) Anmeldetag: **26.02.1997**

(51) Int Cl.6: **G06F 17/18**

(86) Internationale Anmeldenummer:
**PCT/DE97/00338**

(87) Internationale Veröffentlichungsnummer:
**WO 97/33237 (12.09.1997 Gazette 1997/39)**

(54) **VERFAHREN UND VORRICHTUNG ZUR KLASSIFIKATION EINER ZEITREIHE EINES ELEKTRISCHEN SIGNALS DURCH EINEN RECHNER**

METHOD OF AND APPARATUS FOR CLASSIFICATION OF A TIME SERIES OF AN ELECTRICAL SIGNAL BY A COMPUTER,

PROCEDE DE ET APPAREIL POUR CLASSIFICATION D'UNE SERIE CHRONOLOGIQUE D'UN SIGNAL ELECTRIQUE PAR UN ORDINATEUR

(84) Benannte Vertragsstaaten:
**DE FR GB**

(30) Priorität: **06.03.1996 DE 19608734**

(43) Veröffentlichungstag der Anmeldung:
**23.12.1998 Patentblatt 1998/52**

(73) Patentinhaber: **SIEMENS AKTIENGESELLSCHAFT 80333 München (DE)**

(72) Erfinder:
- DECO, Gustavo
  **D-80636 München (DE)**
- SCHÜRMANN, Bernd
  **D-85778 Haimhausen (DE)**

(56) Entgegenhaltungen:
**US-A- 5 625 749**

- **NEURAL COMPUTATION, MARCH 1993, USA, Bd. 5, Nr. 2, ISSN 0899-7667, Seiten 289-304, XP000676836 REDLICH A N: "Redundancy reduction as a strategy for unsupervised learning"**
- **WCNN '95. WORLD CONGRESS ON NEURAL NETWORKS. 1995 INTERNATIONAL NEURAL NETWORK SOCIETY ANNUAL MEETING, PROCEEDINGS OF THE WORLD CONGRESS ON NEURAL NETWORKS, WASHINGTON, DC, USA, 17-21 JULY 1995, ISBN 0-8058-2125-2, 1995, MAHWAH, NJ, USA, LAWRENCE ERLBAUM ASSOCIATES, USA, Seiten 322-325 vol.1, XP000676838 DECO G ET AL: "Unsupervised neural modeling of chaotic time series"**
- **NEURAL COMPUTATION, SEPT. 1993, USA, Bd. 5, Nr. 5, ISSN 0899-7667, Seiten 750-766, XP000676835 REDLICH A N: "Supervised factorial learning"**

## Beschreibung

Die Erfindung betrifft die Ermittlung von nichtlinearen Korrelationen zwischen Abtastwerten einer Zeitreihe und somit die Ermittlung der Dynamik eines Prozesses, wenn von dem Prozeß nur gemessene Daten, die Zeitreihe, gegeben sind. Aus dem Wissen über vorhandene nichtlineare Korrelationen zwischen den Abtastwerten und somit über die Art des Prozeßes, der die Daten generiert, wird die Möglichkeit, grundlegende Fragen wie beispielsweise die Vorhersage zukünftiger Abtastwerte zu beantworten, eröffnet. Dies führt zu der Möglichkeit, ein verlässliches Modell des Prozesses zu entwerfen.

Die Vorhersagbarkeit kann weiterhin verwendet werden als ein Mechanismus zur Auswahl sinnvoller Trainingsdaten aus vorhandenen Daten, den Abtastwerten, zum Training parametrischer Modelle, beispielsweise neuronaler Netze. Weitere Anwendungsbereiche finden sich in der Medizin, wo beispielsweise vorhandene nichlineare Korrelationen zwischen den Abtastwerten eines Elektrokardiogramm-Signals auf eine Gefährdung des Herzens, von dem das Elektrokardiogramm gemessen wurde, bezüglich eines sogenannten plötzlichen Herztodes hinweist. Weiterhin ist dieses Verfahren ebenso als Werkzeug zur Entwicklung von Investitionsstrategien einsetzbar.

Eine sogenannte kumulante Erweiterung einer Wahrscheinlichkeitsverteilung in einem Fourierraum ist bekannt [1].

Weiterhin ist es bekannt, Parameter, die die statistischen Abhängigkeiten zwischen den Abtastwerten beschreiben, mittels kumulanzbasierter Verfahren in Zeitreihen zu extrahieren [2]. Bei diesem Verfahren werden Kumulanten höherer Ordnung zur Extraktion der Parameter berücksichtigt.

Weiterhin sind Verfahren zum Testen von Hypothesen bekannt, die statistische Verteilungen und Surrogate betreffen [3].

Es ist ferner bekannt, daß bei Existenz nichtlinearer Korrelationen oder auch linearer Korrelationen (statistische Abhängigkeit) zwischen Abtastwerten eines Elektrokardiogramm-Signals (EKG) auf eine Gefährdung des Herzens, von dem das Elektrokardiogramm-Signal gemessen wurde, bezüglich des sogenannten plötzlichen Herztodes geschlossen werden kann [4]. Bei diesem Verfahren werden jedoch die nichtlinearen stochastischen Korrelationen zwischen den Abtastwerten einer Zeitreihe empirisch aus einer Betrachtung einer graphischen Darstellung einer Fourier-Transformation der Zeitreihe ermittelt. Dieses Verfahren weist vor allem den Nachteil auf, daß es durch die empirische Vorgehensweise ungenau ist und nur langsam durchführbar ist. Vor allem der Übergang bei der Klassifikation eines Elektrokardiogramm-Signals von einem ungefährdeten Herzen zu einem gefährdeten Herzen ist allein durch die graphische Darstellung der Fourier-Transformierten schwer zu ziehen. Somit kann es zu Fehlklassifikationen der Zeitreihe kommen.

Aus dem Dokument [5] ist es bekannt, die lokale Sauerstoffspannung eines Gehirns zu messen.

Der Erfindung liegt das Problem zugrunde, eine eine vorgebbare Anzahl von Abtastwerten aufweisende Zeitreihe mit Hilfe eines Rechners zu klassifizieren, wobei die Nachteile des im vorigen beschriebenen empirischen Verfahrens vermieden werden sollen.

Das Problem wird durch das Verfahren gemäß Patentanspruch 1 und durch die Vorrichtung gemäß Patentanspruch 13 gelöst.

Bei dem erfindungsgemäßen Verfahren bzw. der erfindungsgemäßen Vorrichtung wird für die Zeitreihe, die eine vorgebbare Anzahl von Abtastwerten aufweist, eine vorgebbare Anzahl von Surrogaten bestimmt. Ferner wird sowohl für die Abtastwerte der Zeitreihe als auch die Abtastwerte der Surrogate mit einer kumulantenbasierten Methode die Existenz nichtlinearer Korrelationen zwischen den Abtastwerten der Zeitreihe bzw. zwischen den Abtastwerten der Surrogaten ermittelt. Aus der Betrachtung der unterschiedlichen Werte für die nichtlinearen Korrelationen zwischen den Abtastwerten der Zeitreihe sowie den nichtlinearen Korrelationen zwischen den Abtastwerten der Surrogaten, wird die Zeitreihe klassifiziert.

Durch diese Vorgehensweise ist eine analytische Klassifikation einer Zeitreihe und somit eine analytische Ermittlung nichtlinearer Korrelationen der Zeitreihe durch einen Rechner möglich. Dies führt zu einer schnellen Durchführbarkeit des Verfahrens sowie zu verlässlicheren Klassifikationsergebnissen, die das Verfahren liefert.

Weiterbildungen des erfindungsgemäßen Verfahrens bzw. der erfindungsgemäßen Vorrichtung ergeben sich aus den abhängigen Ansprüchen.

Es ist vorteilhaft, ausgehend von einem Bezugszeitpunkt nur eine vorgebbare Anzahl von Abtastwerten, die zeitlich vor dem Bezugszeitpunkt liegen, innerhalb des Verfahrens zu berücksichtigen. Durch diese Vorgehensweise, daß nur ein Teil der Abtastwerte der Zeitreihe und nicht alle Abtastwerte der Zeitreihe betrachtet werden, ist es bei dieser Weiterbildung des Verfahrens nicht notwendig, eine Stationarität der Art der nichtlinearen Korrelation zwischen den Abtastwerten für die gesamte Zeitreihe vorauszusetzen. Dadurch wird die Genauigkeit des erfindungsgemäßen Verfahrens weiter erhöht.

Ferner ist es vorteilhaft, die Klassifikation nur in "binärer Form" durchzuführen, daß heißt bei der Klassifikation nur zwischen einem ersten Zeitreihentyp und einem zweiten Zeitreihentyp zu unterscheiden, je nachdem, ob eine aus dem Unterschied der nichtlinearen Korrelationen der Abtastwerte der Zeitreihe und der Abtastwerte der Surrogate gebildete Signifikanz über einer vorgebbaren Schranke liegt. Durch diese binäre Klassifikation wird die Durchführung des Verfahrens erheblich beschleunigt, da bei der Durchführung durch einen Rechner weniger Klassifikationsparameter berücksichtigt werden müssen.

Die Genauigkeit der Ergebnisse des erfindungsgemäßen Verfahrens kann dadurch erhöht werden, daß zur Ermittlung der nichtlinearen Korrelationen Kumulanten von Momenten höherer als zweiter Ordnung berücksichtigt werden.

Ferner ist es vorteilhaft, daß das Verfahren auf eine Zeitreihe angewendet wird, welches durch ein Elektrokardiogramm-Signal (EKG) gegeben ist. Hierdurch wird es bei der Klassifikation möglich, ein gefährdetes Herz bezüglich des plötzlichen Herztodes früh zu erkennen, womit dem behandelnden Arzt die Möglichkeit gegeben wird, frühzeitig Gegenmaßnahmen gegen den plötzlichen Herztod für den Patienten einzuleiten.

Eine vorteilhafte Verwendung des erfindungsgemäßen Verfahrens liegt in der Auswahl von Trainingsdaten für ein neuronales Netz. Hierbei können Abtastwerte, zwischen denen keine bzw. fast keine nichtlinearen Korrelationen (statistische Abhängigkeiten) ermittelt wurden, als Trainingsdaten vernachlässigt werden. Damit werden nur die Abtastwerte als Trainingsdaten für ein neuronales Netz verwendet, welche tatsächlich auch nichtlineare Korrelationen (statistische Abhängigkeiten) aufweisen, also für das Training eine sinnvolle Aussagekraft bezüglich eines Trainingsziels aufweisen.

Ein Ausführungsbeispiel ist in den Figuren dargestellt und wird im weiteren näher erläutert.

Es zeigen

Figur 1 ein Ablaufdiagramm, in dem die einzelnen Verfahrensschritte dargestellt sind;

Figur 2 ein Ablaufdiagramm, welches die Weiterbildung des erfindungsgemäßen Verfahrens mit einer Klassifikation in nur zwei Zeitreihentypen, beschreibt;

Figur 3 ein Blockdiagramm, in dem verschiedene Möglichkeiten dargestellt sind, von welcher Art die Zeitreihe beispielsweise sein kann;

Figur 4 eine Skizze, in der ein Rechner, mit dem das Verfahren durchgeführt wird, dargestellt ist.

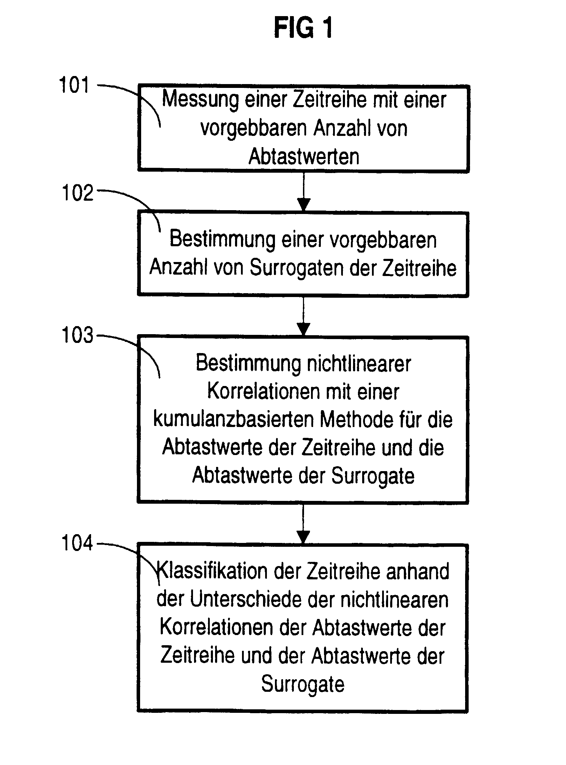

In Figur 1 sind die einzelnen Verfahrensschritte des erfindungsgemäßen Verfahrens dargestellt. In einem ersten Schritt 101 wird eine Zeitreihe gemessen. Die Zeitreihe kann analoger Art sein, womit eine Abtastung der Zeitreihe nötig wird, damit die Zeitreihe in einem Rechner R verarbeitet werden kann. Liegt die Zeitreihe jedoch schon in einzelnen digitalen Werten vor, so ist eine Analog/Digitalwandlung der Zeitreihe nicht mehr erforderlich. Welche Art von Signalen die Zeitreihe beispielsweise repräsentieren kann, wird im weiteren erläutert. Die Zeitreihe liegt digital vor, d. h. sie weist eine vorgebbare, abhängig von einem Abtastintervall, mit dem die Zeitreihe abgetastet wird, Anzahl von Abtastwerten auf.

In einem zweiten Schritt 102 wird eine vorgebbare Anzahl von Surrogaten $S_i$ bestimmt. Hierbei ist ein Index i eine natürliche Zahl zwischen 1 und N, wobei mit N eine Anzahl der Surrogaten $S_i$ bezeichnet wird. Verfahren zur Bestimmung von Surrogaten und deren Bedeutung sind in dem Dokument [3] beschrieben. Unter Surrogaten sind im Rahmen dieses Dokumentes Zeitreihen zu verstehen, die aus der gemessenen Zeitreihe gebildet werden beispielsweise durch einfaches zeitliches Vertauschen der Reihenfolge der Abtastwerte der Zeitreihe, ohne die Werte der Abtastwerte zu verändern. Dies bedeutet, daß die Surrogate $S_i$ einige gegebene Eigenschaften der Zeitreihe aufweisen, beispielsweise die gleiche gesamte Werteverteilung der Abtastwerte, und dennoch konsistent sind mit einer zu testenden, im weiteren beschrieben Hypothese, die im weiteren als Null-Hypothese H bezeichnet wird. Durch die Bildung der Surrogate $S_i$, vor allem durch die Änderung der Reihenfolge der Abtastwerte der Zeitreihe werden allerdings nichtlineare Korrelationen oder lineare Korrelationen (statistische Abhängigkeit), die die Abtastwerte der Zeitreihe eventuell aufweisen, für die Surrogate $S_i$ "zerstört".

Mit der Null-Hypothese H wird im Rahmen des erfindungsgemäßen Verfahrens eine Vorhersagbarkeit zukünftiger Abtastwerte bezüglich eines Bezugszeitpunktes, abhängig von vergangenen Abtastwerten bezeichnet. Die Null-Hypothese H ergibt sich aus der Überprüfung der statistischen Unabhängigkeit zwischen den einzelnen Abtastwerten der Zeitreihe bzw. der Abtastwerte der Surrogaten.

Die Null-Hypothese H ergibt sich demnach aus:

$$H = P(x_1, x_2, ..., x_m) = P(x_1)P(x_2,..., x_m) \qquad (1).$$

Hierbei bezeichnet $P(x_1, x_2, ..., x_m)$ eine Verbundwahrscheinlichkeit, die die Wahrscheinlichkeit angibt, daß eine Folge von m Abtastwerten die Werte $x_1, x_2 ... x_m$ aufweisen. Mit $P(x_1)$ wird die Wahrscheinlichkeit bezeichnet, daß zu einem Bezugszeitpunkt 1 der Abtastwert der Zeitreihe den Wert $x_1$ aufweist. Entsprechend kennzeichnet die Verbundwahrscheinlichkeit $P(x_2,..., x_m)$ die Verbundwahrscheinlichkeit für das Auftreten der Abtastwerte $x_2, ..., x_m$.

Mit $\phi(K_1, K_2,..., K_m)$ wird eine Fourier-Transformierte der Null-Hypothese H bezeichnet die sich ergibt aus:

$$\Phi(K_1, K_2, \ldots, K_m) = dx_1 dx_2 \ldots dx_m e^{i\left(\sum_{j=1}^{m} x_j K_j\right)} P(x_1, x_2, \ldots, x_m \tag{2}.$$

Mit $K_1, K_2,..., K_m$ werden die Variablen der Funktion $\phi(K_1, K_2,..., K_m)$ im Fourier-Raum bezeichnet.

Bei erfüllter Gleichung (1) gilt entsprechend in dem Fourier-Raum:

$$\Phi(K_1, K_2,..., K_m) = \varphi(K_1)\lambda(K_2,..., K_m) \tag{3}.$$

Die Gleichung (3) gibt eine Bedingung für die statistische Unabhängigkeit der Abtastwerte der Zeitreihe im Fourierraum entsprechend der Gleichung (1) der Null-Hypothese H im Zeitbereich an.

Hierbei sind die einzelnen Komponenten der Fourier-Transformierten $\varphi(K_1)$ und $\lambda(K_2,..., K_m)$ gegeben durch

$$\varphi(K_1) = dx_1 e^{i \cdot K_1 x_1} P(x_1 \tag{4}$$

und

$$\lambda(K_2, \ldots, K_m) = dx_2 \ldots dx_m e^{i\left(\sum_{j=2}^{m} x_j K_j\right)} P(x_2, \ldots, x_m \tag{5}.$$

Durch Logarithmieren der Gleichung (3) ergibt sich

$$\ln(\Phi(K_1, K_2,..., K_m)) = \ln(\varphi(K_1)) + \ln(\lambda(K_2,...,K_m)) \tag{6}.$$

Durch Kumulantenentwicklung der Gleichung (6), die aus dem Dokument [1] bekannt ist, ergibt sich nach einigen Umformungen eine Bedingung für die statistische Unabhängigkeit der Abtastwerte der Zeitreihe:

$$\sum_{n=1}^{\infty} \frac{i^n}{n!} \sum_{i_1,\ldots,i_n=1}^{m} N_{i_1 \ldots i_n} \cdot K_{i_1} \ldots K_{i_n} = \tag{7}.$$
$$= \sum_{n=1}^{\infty} \frac{i^n}{n!} \cdot N_1^{(n)} \cdot K_1^n +$$
$$+ \sum_{n=1}^{\infty} \frac{i^n}{n!} \sum_{i_1,\ldots,i_n=2}^{m} N_{i_1 \ldots i_n} \cdot K_{i_1} \ldots K_{i_n}$$

Hierbei werden mit $i_1...i_n$ jeweils multidimensionale Kumulanten der Ordnung n bezeichnet. Mit $N_1^{(n)}$ werden eindimensionale Kumulanten der Ordnung n bezeichnet.

Daraus ergibt sich:

$$\sum_{n=1}^{\infty} \frac{i^n}{n!} \sum_{i_2,\ldots,i_n=1}^{m} \aleph_{1 i_2 \ldots i_n} \cdot 1 - \delta_{1 i_2 \ldots i_n} \cdot K_{i_2} \ldots K_{i_n} \qquad (8)$$

Hierbei ist mit $\delta_{ij\ldots l}$ ein sogenanntes Kronecker-Delta bezeichnet, für welches gilt:

$$\begin{aligned} \delta_{ij\ldots l} &= 1 & \text{für } i = j = \ldots = l \\ \delta_{ij\ldots l} &= 0 & \text{sonst} \end{aligned} \qquad (9).$$

Da die Gleichung (3) für alle Vektoren $\underline{K}$ gelten soll, müssen alle Koeffizienten jeder Summe der Gleichung (8) den Wert 0 aufweisen. Dies bedeutet, daß die nichtdiagonalen Elemente aller Kumulanten, deren erster Index den Wert 1 aufweist, den Wert 0 aufweisen. Somit ergibt sich:

$$\aleph_{1 i_2 \ldots i_n} \qquad \text{für } 1 \neq i_2 \text{ oder } 1 \neq i_3 \text{ oder } 1 \neq i_n \qquad (10)$$

als Bedingung für die statistische Unabhängigkeit der Abtastwerte.

Als Maß, welches die statistische Unabhängigkeit der Zeitreihe angibt, wird im folgenden eine Kostenfunktion D verwendet, die sich ergibt aus

$$D = \sum_{n=1}^{\infty} \sum_{i_2,\ldots,i_n=1}^{m} (\aleph_{1 i_2 \ldots i_n} \cdot (1 - \delta_{1 i_2 \ldots i_n}))^2 \qquad (11).$$

Die Kostenfunktion D weist immer einen Wert auf, der größer ist als 0. Durch die Kostenfunktion D wird der Grad der statistischen Abgängigkeit zwischen den Abtastwerten der Zeitreihe angegeben, was existierenden nichtlinearen Korrelationen zwischen den Abtastwerten der Zeitreihe entspricht. Quantitativ bedeutet dies, daß, je kleiner der Wert der Kostenfunktion D wird, desto geringer werden die nichtlinearen Korrelationen zwischen den Abtastwerten der Zeitreihe und je größer der Wert der Kostenfunktion D wird, desto größere nichtlineare Korrelationen zwischen den Abtastwerten der Zeitreihe und den Abtastwerten der Surrogaten sind somit vorhanden.

Bei der Ermittlung der nichtlinearen Korrelationen können Kumulanten von Momenten unterschiedlicher Ordnungen berücksichtigt werden. Im weiteren werden ohne Einschränkung der Allgemeingültigkeit lediglich Momente von Kumulanten bis zur vierten Ordnung berücksichtigt. Kumulanten mit Momenten zweiter Ordnung entsprechen hierbei den einzelnen Matrixelementen einer Covarianzmatrix, die, wie jedem Fachmann geläufig, lineare Korrelationen zwischen den Abtastwerten der Zeitreihe beschreiben.

Momente der Kumulanten erster Ordnung ergeben sich aus:

$$\aleph_i = 0 \qquad (12).$$

Momente der Kumulanten zweiter Ordnung ergeben sich aus:

$$\aleph_{ij} = C_{ij} \qquad (13).$$

Hierbei werden mit $C_{ij}$ Matrixelemente einer Covarianzmatrix bezeichnet.

Momente der Kumulanten dritter Ordnung ergeben sich aus:

$$\aleph_{ijk} = C_{ijk} \qquad (14).$$

Momente vierter Ordnung der Kumulanten ergeben sich aus:

$$\aleph_{ijkl} = C_{ijkl} - C_{ij} \cdot C_{kl} - C_{ik} \cdot C_{lj} - C_{il} \cdot C_{jk} \qquad (15).$$

Hierbei sind jeweils mit $C_{i...j}$ Momententensoren für die Kumulanten entsprechender Ordnung gegeben, die sich nach folgender Vorschrift bestimmen:

$$C_{i...j} = dx_i \ ... \ dx_j \cdot P(x_i...x_j)x_i \cdots x_j \qquad (16).$$

Die Bestimmung der Kostenfunktion D wird sowohl für die Zeitreihe als auch für die vorgegebene Anzahl von Surrogaten $S_i$ durchgeführt 103.

Anhand der Ergebnisse der Kostenfunktion D für die Zeitreihe $D_0$ sowie der Kostenfunktion D für die Surrogaten $D_{Si}$ wird eine Klassifikation der Zeitreihe durchgeführt. Die Unterschiede in der Ergebnissen der Kostenfunktion D repräsentieren die Unterschiede der nichtlinearen Korrelationen zwischen den Abtastwerten der Zeitreihe sowie die nichtlinearen Korrelationen der Abtastwerte der Surrogate $S_i$. Der Vergleich kann auf unterschiedliche Weise erfolgen:

Eine Möglichkeit liegt beispielsweise darin, eine Signifikanz S zu ermitteln 201, die sich ergibt aus:

$$S = \frac{|D_0 - \mu_S|}{\sigma_S} \qquad (17).$$

Hierbei bezeichnet $\mu_S$ einen Durchschnittswert über die ermittelten Ergebnisse der Kostenfunktion D, die auf die Surrogate $S_i$ angewendet wurden. Der Durchschnittswert $\mu_S$ ergibt sich beispielsweise aus

$$\mu_S = \frac{1}{N} \sum_{i=1}^{N} D_{Si} \qquad (18).$$

Mit $\sigma_S$ wird eine Varianz über die Ergebnisse der Kostenfunktion D für die Surrogate $S_i$ gekennzeichnet. Sie ergibt sich beispielsweise aus

$$\sigma_S = \sqrt{\frac{1}{N} \sum_{i=1}^{N} (D_{Si} - \mu_S)^2} \qquad (19).$$

Dies Vorgehensweise ist jedoch in keinster Weise als alleinig mögliche Vorgehensweise im Rahmen dieses Verfahrens zu verstehen. Weitere Möglichkeiten, die Ergebnisse der Kostenfunktion der Zeitreihe und die Ergebnisse der Kostenfunktion für die Surrogate $D_{Si}$ miteinander zu vergleichen, sind dem Fachmann geläufig und können ohne Einschränkung in dem erfindungsgemäßen Verfahren verwendet werden.

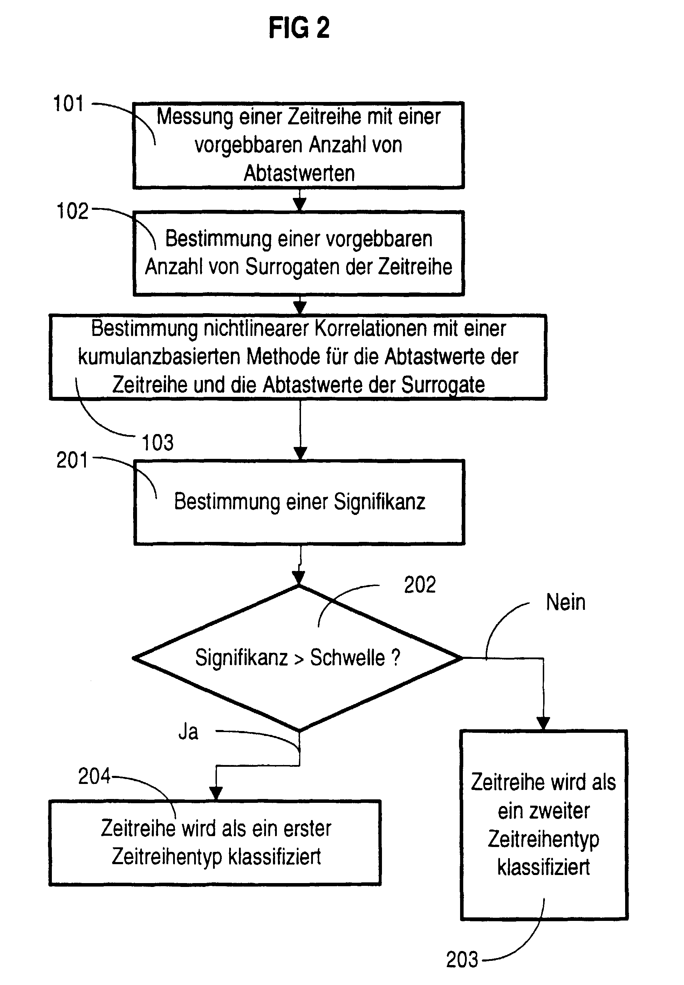

In einer Weiterbildung der Verfahrens (vgl. Figur 2) ist es vorteilhaft, eine "binäre Klassifikation" durchzuführen, d. h., die Klassifikation darin besteht, die Zeitreihe in einen ersten Zeitreihentyp und in einen zweiten Zeitreihentyp einzuordnen. In anderen Worten wird auf diese Weise eine eventuell vorhandene statistische Abhängigkeit zwischen den Abtastwerten detektiert. Hierbei wird, wie in Figur 2 dargestellt, anhand beispielsweise der auf die in der vorigen Weise beschriebenen bestimmten Signifikanz S 201 geprüft, ob der Wert der Signifikanz S größer ist als eine vorgebbare Schranke 202. Die Schranke ist je nach Anwendungsziel und Genauigkeit bzw. Verlässlichkeit des Ergebnisses durch den Benutzer vorgebbar. Ist die Signifikanz S größer als die vorgebbare Schranke, so wird die Zeitreihe als ein erster Zeitreihentyp klassifiziert 204. Ist die Signifikanz S kleiner als die vorgebbare Schranke, so wird die Zeitreihe als ein zweiter Zeitreihentyp klassifiziert 203. Durch diese einfachste Art der Klassifikation wird das Verfahren, welches notwendigerweise mit einem Rechner durchgeführt wird, beschleunigt, da nur eine Art von Parameter untersucht werden muß zur Klassifikation der Zeitreihe.

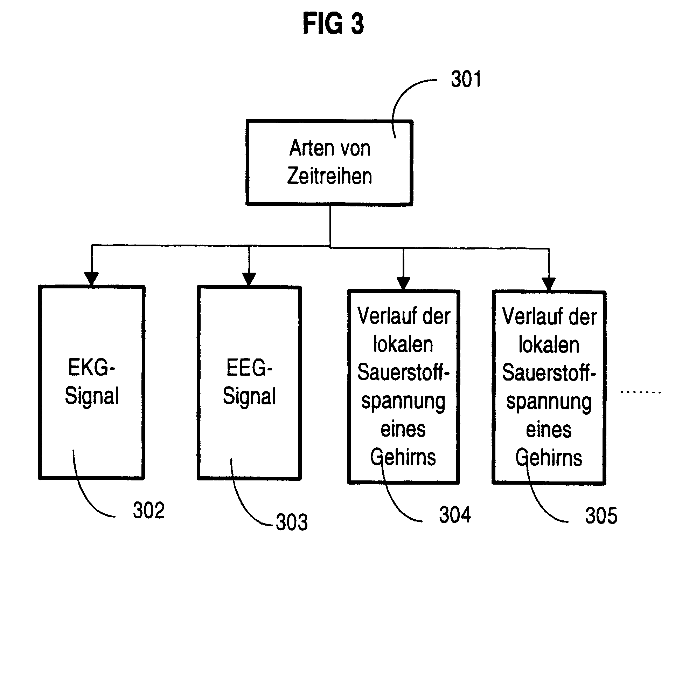

In Figur 3 sind verschiedene Arten von Signalen, die die Zeitreihe realisieren können, dargestellt 301. Die Zeitreihe kann durch ein Elektrokardiogramm-Signal (EKG) realisiert sein. Für diesen Anwendungsfall ist eine vorteilhafte Verwendung vorgesehen, da, wie in dem Dokument [4] beschrieben ist, für ein Herz bei Auftreten nichtlinearer Korrelationen zwischen den Abtastwerten des Elektrokardiogramm-Signals darauf geschlossen werden kann, daß dieses Herz gefährdet ist bezüglich des Eintretens eines Plötzlichen Herztodes. Demnach entspricht bei der binären Klassifikation 202, 203, 204, die Klassifikation der Zeitreihe in den ersten Zeitreihentyp 204 einer Klassifikation des Elektrokardiogramm-Signals in ein Elektrokardiogramm-Signal eines bezüglich des Plötzlichen Herztodes gefährdeten Herzens. Der zweite Zeitreihentyp entspricht einem Elektrokardiogramm-Signal eines bezüglich des Plötzlichen Herztodes nicht gefährdeten Herzens.

Ferner ist es vorgesehen, daß die Zeitreihe durch ein Elektroencephalogramm-Signal (EEG) gegeben ist 303.

Weiterhin kann die Zeitreihe durch ein Signal gegeben sein, welches den Verlauf einer lokalen Sauerstoffspannung eines Gehirns beschreibt 304.

Weiterhin kann die Zeitreihe durch ein Signal gegeben sein, welches variable Kurse eines Finanzmarktes, beispielsweise im Devisenhandel oder allgemeine Aktienkurse, Kurse von Aktienindizes, usw. beschreiben, realisiert sein 305.

In einer vorteilhaften Weiterbildung des Verfahrens ist es vorgesehen, nicht alle Abtastwerte der Zeitreihe zu berücksichtigen, sondern nur eine vorgebbare Anzahl. Durch diese Weiterbildung ist eine Annahme einer Stationarität der Struktur der nichtlinearen Korrelationen zwischen den Abtastwerten für die gesamten Abtastwerte der Zeitreihe nicht mehr erforderlich.

Weiterhin liegt eine vorteilhafte Verwendung des erfindungsgemäßen Verfahrens darin, daß für den Fall, daß die Zeitreihe Abtastwerte aufweist, die als Trainingsdaten für ein neuronales Netz in einer beliebigen Anwendung vorgesehen sind, eine Auswahl der Trainingsdaten aus den Abtastwerteb stattfinden kann. Da vorhandene nichtlineare Korrelationen, also statistische Abhängigkeiten zwischen den Abtastwerten für den Fall, daß die Abtastwerte Trainingsdaten eines neuronalen Netzes sind, bedeutet, daß diese bezüglich eines zu trainierenden Lernzieles Aussagekraft besitzen und diejenigen Abtastwerte, die statistisch voneinander unabhängig sind, als Trainingsdaten bezüglich des Trainingsziels keinen Informationsgehalt aufweisen, also als Trainingsdaten sinnlos sind. Damit kann der Trainingsdatensatz reduziert werden, wenn man Abtastwerte, die keine nichtlinearen Korrelationen aufweisen, nicht als Trainingsdaten verwendet. Dadurch wird der Aufwand und die Dauer eines Trainings eines neuronalen Netzes erheblich verkürzt, ohne den Informationsgehalt der Trainingsdaten zu reduzieren.

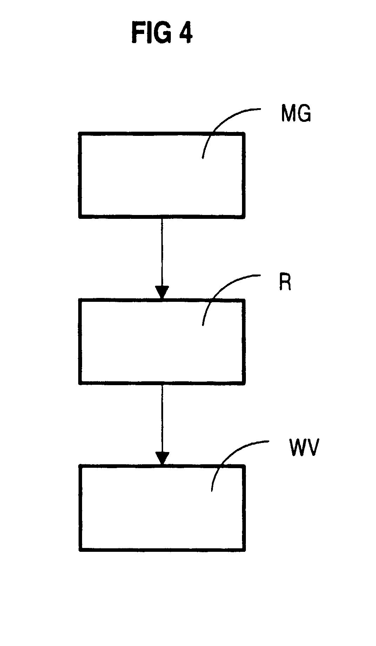

In Figur 4 ist der Rechner R dargestellt, mit dem das erfindungsgemäße Verfahren notwendigerweise durchgeführt wird.

Der Rechner R verarbeitet die von dem Meßgerät MG aufgenommenen, und dem Rechner R zugeführten Zeitreihen.

Hierbei ist es nicht von Bedeutung, ob die Bildung der Abtastwerte aus dem möglicherweise analogen Signal in dem Meßgerät MG oder in dem Rechner R durchgeführt wird. Beide Varianten sind für das erfindungsgemäße Verfahren vorgesehen.

Das Meßgerät MG kann beispielsweise ein Elektrokardiograph (EKG), ein Elektroencepahlograph (EEG) oder auch ein Gerät sein, welches nach dem in [2] dargestellten Verfahren arbeitet.

Das Klassifikationsergebnis, welches durch den Rechner R auf die im vorigen beschriebene Weise ermittelt wird, wird in einem Mittel zur Weiterverarbeitung WV weiterverarbeitet, beispielsweise einem Benutzer dargestellt. Dieses Mittel WV kann beispielsweise ein Drucker, ein Bildschirm oder auch ein Lautsprecher sein, über das ein akustisches oder visuelles Signal an einen Benutzer weitergegeben wird.

Im Rahmen dieses Dokuments wurden folgende Veröffentlichungen zitiert:

[1] C. Gardiner, Handbook of Stochastic Methods, 2. Auflage, Springer-Verlag, ISBN 0-387-11357-6, S. 33 - 36, New York, 1985

[2] G. Deco und B. Schürmann, Learning Time Series Evolution by Unsupervised Extraction of Correlations, Physical Review E, Vol. 51, No. 3, S. 1780 - 1790, März 1995

[3] L Breiman, Statistics, Houghton Mifflin Company, Boston, S. 254 - 284, 1973,

[4] G. Morfill, Komplexitätsanalyse in der Kardiologie, Physikalische Blätter, Vol. 50, Nr. 2, S. 156 bis 160, 1994

[5] LICOX, GMS, Gesellschaft für Medizinische Sondentechnik mbH, Advanced Tissue Monitoring

**Patentansprüche**

1. Verfahren zur Klassifikation einer Zeitreihe eines elektrischen Signals, die eine vorgebbare Anzahl von Abtastwerten aufweist, durch einen Rechner,

   - bei dem für die Zeitreihe eine vorgebbare Anzahl von Surrogaten ($S_i$, i=1..N) bestimmt wird (102),
   - bei dem für die Zeitreihe und für die Surrogate ($S_i$) nichtlineare Korrelationen und/oder lineare Korrelationen (statistische Abhängigkeit) zwischen den Abtastwerten der Zeitreihe ($D_0$) und nichtlineare Korrelationen zwischen den Abtastwerten der Surrogate ($D_{Si}$) mit einer kumulantenbasierte Methode ermittelt werden (103),
   - bei dem aus den Unterschieden der nichtlinearen Korrelationen und/oder linearen Korrelationen der Abtastwerte der Zeitreihe ($D_0$) und der nichtlinearen Korrelationen und/oder linearen Korrelationen der Abtastwerte der Surrogate ($D_{Si}$) die Zeitreihe klassifiziert wird (104).

2. Verfahren nach Anspruch 1, bei dem aus den Unterschieden der nichtlinearen Korrelationen und/oder linearen Korrelationen der Abtastwerte der Zeitreiheund der nichtlinearen Korrelationen und/oder linearen Korrelationen der Abtastwerte der Surrogate ($D_{Si}$) eine Signifikanz (S) ermittelt wird nach der Vorschrift

$$S = \frac{|D_0 - \mu_S|}{\sigma_S}$$

   wobei mit

   $\mu_S$ ein Durchschnittswert der nichtlinearen Korrelationen der Abtastwerte der Surrogate ($D_{Si}$) bezeichnet wird,
   $\sigma_S$ eine Varianz der nichtlinearen Korrelatione der Abtastwerte der Surrogate ($D_{Si}$) bezeichnet wird.

3. Verfahren nach Anspruch 2, bei dem der Durchschnittswert ($\mu_S$) der nichtlinearen Korrelationen und/oder linearen Korrelationen der Abtastwerte der Surrogate ($D_{Si}$) bestimmt wird nach der Vorschrift

$$\mu_S = \frac{1}{N} \sum_{i=1}^{N} D_{Si} \, .$$

4. Verfahren nach Anspruch 2 oder 3, bei dem die Varianz der nichtlinearen Korrelationen und/oder linearen Korrelationen der Abtastwerte der Surrogate ($D_{Si}$) bestimmt wird nach der Vorschrift

$$\sigma_S = \sqrt{\frac{1}{N} \sum_{i=1}^{N} (D_{Si} - \mu_S)^2} \, .$$

5. Verfahren nach einem der Ansprüche 1 bis 4, bei dem die Klassifikation durchgeführt wird, indem die Zeitreihe in einen ersten Zeitreihentyp, mit dem eine vorhandene stochastische Struktur zwischen den Abtastwerten der Zeitreihe beschrieben wird, oder einen zweiten Zeitreihentyp, mit dem nicht vorhandene stochastische Strukturen zwischen den Abtastwerten der Zeitreihe beschrieben wird.

6. Verfahren nach einem der Ansprüche 2 bis 5, bei dem die Zeitreihe als der erste Zeitreihentyp klassifiziert wird, wenn die Signifikanz (S) einen Wert aufweist, der größer ist als eine vorgebbare Schranke.

7. Verfahren nach einem der Ansprüche 1 bis 6, bei dem bei der kumulantenbasierte Methode zur Ermittlung der nichtlinearen Korrelationen Kumulanten von Momenten mindestens zweiter Ordnung berücksichtigt werden.

8. Verfahren nach einem der Ansprüche 1 bis 7, bei dem von der Zeitreihe nur eine vorgebbare Anzahl von Abtastwerten, die zeitlich vor einem Bezugszeitpunkt liegen, berücksichtigt werden.

9. Verfahren nach einem der Ansprüche 1 bis 8, bei dem die Zeitreihe ein gemessenes ElektrokardiogrammSignal (EKG) ist.

10. Verfahren nach einem der Ansprüche 1 bis 8, bei dem die Zeitreihe ein gemessenes Elektroencephalogramm-Signal (EEG) ist.

11. Verfahren nach einem der Ansprüche 1 bis 8, bei dem die Zeitreihe ein gemessenes Signal ist, durch das ein Verlauf eines Sauerstoffdrucks eines Gehirns beschrieben wird, ist.

12. Verwendung des Verfahrens nach einem der Ansprüche 1 bis 12 zur Auswahl von Trainingsdaten für ein neuronales Netzaus aus den Abtastwerten der Zeitreihe, bei dem nur die Abtastwerten als Trainingsdaten verwendet werden, die nichtlineare Korrelationen und/oder lineare Korrelationen aufweisen.

13. Vorrichtung zur Klassifikation einer Zeitreihe eines elektrischen Signals, die eine vorgebbare Anzahl von Abtastwerten aufweist, mit einem Prozessor, der derart eingerichtet ist, daß folgende Schritte durchführbar sind:

 - für die Zeitreihe wird eine vorgebbare Anzahl von Surrogaten $(S_i, i=1..N)$ bestimmt,
 - für die Zeitreihe und für die Surrogate $(S_i)$ werden nichtlineare Korrelationen und/oder lineare Korrelationen (statistische Abhängigkeit) zwischen den Abtastwerten der Zeitreihe $(D_0)$ und nichtlineare Korrelationen zwischen den Abtastwerten der Surrogate $(D_{Si})$ mit einer kumulantenbasierte Methode ermittelt,
 - aus den Unterschieden der nichtlinearen Korrelationen und/oder linearen Korrelationen der Abtastwerte der Zeitreihe $(D_0)$ und der nichtlinearen Korrelationen und/oder linearen Korrelationen der Abtastwerte der Surrogate $(D_{Si})$ wird die Zeitreihe klassifiziert.

14. Vorrichtung nach Anspruch 13, bei der der Prozessor derart eingerichtet ist, daß aus den Unterschieden der nichtlinearen Korrelationen und/oder Linearen Korrelationen der Abtastwerte der Zeitreihe und der nichtlinearen Korrelationen und/oder linearen Korrelationen der Abtastwerte der Surrogate $(D_{Si})$ eine Signifikanz (S) ermittelt wird nach der Vorschrift

$$S = \frac{|D_0 - \mu_S|}{\sigma_S}$$

wobei mit

$\mu_S$ ein Durchschnittswert der nichtlinearen Korrelationen der Abtastwerte der Surrogate $(D_{Si})$ bezeichnet wird, $\sigma_S$ eine Varianz der nichtlinearen Korrelationen der Abtastwerte der Surrogate $(D_{Si})$ bezeichnet wird.

15. Vorrichtung nach Anspruch 14, bei der der Prozessor derart eingerichtet ist, daß der Durchschnittswert $(\mu_S)$ der nichtlinearen Korrelationen und/oder linearen Korrelationen der Abtastwerte der Surrogate $(D_{Si})$ bestimmt wird nach der Vorschrift

$$\mu_S = \frac{1}{N} \sum_{i=1}^{N} D_{Si}.$$

16. Vorrichtung nach Anspruch 14 oder 15, bei der der Prozessor derart eingerichtet ist, daß die Varianz der nichtlinearen Korrelationen und/oder linearen Korrelationen der Abtastwerte der Surrogate ($D_{Si}$) bestimmt wird nach der Vorschrift

$$\sigma_S = \sqrt{\frac{1}{N} \sum_{i=1}^{N} (D_{Si} - \mu_S)^2} \, .$$

17. Vorrichtung nach einem der Ansprüche 13 bis 16, bei der der Prozessor derart eingerichtet ist, daß die Klassifikation durchgeführt wird, indem die Zeitreihe in einen ersten Zeitreihentyp, mit dem eine vorhandene stochastische Struktur zwischen den Abtastwerten der Zeitreihe beschrieben wird, oder einen zweiten Zeitreihentyp, mit dem nicht vorhandene stochastische Strukturen zwischen den Abtastwerten der Zeitreihe beschrieben wird.

18. Vorrichtung nach einem der Ansprüche 14 bis 17, bei der der Prozessor derart eingerichtet ist, daß die Zeitreihe als der erste Zeitreihentyp klassifiziert wird, wenn die Signifikanz (S) einen Wert aufweist, der größer ist als eine vorgebbare Schranke.

19. Vorrichtung nach einem der Ansprüche 13 bis 18, bei der der Prozessor derart eingerichtet ist, daß bei der kumulantenbasierte Methode zur Ermittlung der nichtlinearen Korrelationen Kumulanten von Momenten mindestens zweiter Ordnung berücksichtigt werden.

20. Vorrichtung nach einem der Ansprüche 13 bis 19, bei der der Prozessor derart eingerichtet ist, daß von der Zeitreihe nur eine vorgebbare Anzahl von Abtastwerte, die zeitlich vor einem Bezugszeitpunkt liegen, berücksichtigt werden.

21. Vorrichtung nach einem der Ansprüche 13 bis 20, bei der die Zeitreihe ein gemessenes Elektrokardiogramm-Signal (EKG) ist.

22. Vorrichtung nach einem der Ansprüche 13 bis 20, bei der die Zeitreihe ein gemessenes Elektroencephalogramm-Signal (EEG) ist.

23. Vorrichtung nach einem der Ansprüche 13 bis 20, bei der die Zeitreihe ein gemessenes Signal ist, durch das ein Verlauf eines Sauerstoffdrucks eines Gehirns beschrieben wird, ist.

24. Verwendung der Vorrichtung nach einem der Ansprüche 13 bis 23 zur Auswahl von Trainingsdaten für ein neuronales Netz aus den Abtastwerten der Zeitreihe, bei dem nur die Abtastwerten als Trainingsdaten verwender werden, die nichtlineare Korrelationen und/oder lineare Korrelationen aufweisen.

**Claims**

1. Method for classifying a time series of an electrical signal, which contains a predeterminable number of sample values, using a computer,

   - in which a predeterminable number of surrogates (Si, i=1..N) are determined (102) for the time sequence,
   - in which, for the time series and for the surrogates ($S_i$), nonlinear correlations and/or linear correlations (statistical dependence) between the sample values of the time series ($D_0$) and nonlinear correlations between the sample values of the surrogates ($D_{Si}$) are calculated using a cumulant-based method (103),
   - in which the time series is classified (104) from the differences of the nonlinear correlations and/or linear correlations of the sample values of the time series ($D_0$) and the nonlinear correlations and/or linear correlations of the sample values of the surrogates ($D_{Si}$).

2. Method according to Claim 1, in which a significance (S) is calculated from the differences of the nonlinear correlations and/or linear correlations of the sample values of the time series and the nonlinear correlations and/or linear correlations of the sample values of the surrogates ($D_{Si}$), acccrding to the rule

$$S = \frac{|D_0 - \mu_S|}{\sigma_S}$$

in which

$\mu_S$ denotes a mean value of the nonlinear correlations of the sample values of the surrogates ($D_{Si}$),
$\sigma_S$ denotes a variance of the nonlinear correlations of the sample values of the surrogates ($D_{Si}$).

3. Method according to Claim 2, in which the mean value ($\mu_S$) of the nonlinear correlations and/or linear correlations of the sample values of the surrogates ($D_{Si}$) is determined according to the rule

$$\mu_S = \frac{1}{N} \sum_{i=1}^{N} D_{Si} \; .$$

4. Method according to Claim 2 or 3, in which the variance of the nonlinear correlations and/or linear correlations of the sample values of the surrogates ($D_{Si}$) is determined according to the rule

$$\sigma_S = \sqrt{\frac{1}{N} \sum_{i=1}^{N} (D_{Si} - \mu_S)^2} \; .$$

5. Method according to one of Claims 1 to 4, in which the classification is carried out by [lacuna] the time series in a first time series type, by which an existing stochastic structure between the sample values of the time series is described, or a second time series type, by which non-existent stochastic structures between the sample values of the time series are described.

6. Method according to one of Claims 2 to 5, in which the time series is classified as the first time series type if the significance(S) has a value which is greater than a predeterminable limit.

7. Method according to one of Claims 1 to 6, in which moments of at least second order are taken into account in the cumulant-based method for calculating the nonlinear correlations.

8. Method according to one of Claims 1 to 7, in which only a predeterminable number of sample values of the time series, which lie chronologically before a reference time, are taken into account.

9. Method according to one of Claims 1 to 8, in which the time series is a measured electrocardiogram (ECG).

10. Method according to one of Claims 1 to 8, in which the time series is a measured electroencephalogram (EEG).

11. Method according to one of Claims 1 to 8, in which the time series is a measured signal by which an oxygen pressure profile of a brain is described.

12. Use of the method according to one of Claims 1 to 12 to select training data for a neural network from the sample values of the time series, in which only the sample values which have nonlinear correlations and/or linear correlations are used as training data.

13. Device for classifying a time series of an electrical signal, which contains a predeterminable number of sample values, having a processor which is configured in such a way that the following steps can be carried out:

- a predeterminable number of surrogates (Si, i=1..N) are determined for the time sequence,
- for the time series and for the surrogates (Si), nonlinear correlations and/or linear correlations (statistical dependence) between the sample values of the time series ($D_0$) and nonlinear correlations between the sample values of the surrogates ($D_{Si}$) are calculated using a cumulant-based method,

- the time series is classified from the differences of the nonlinear correlations and/or linear correlations of the sample values of the time series ($D_0$) and the nonlinear correlations and/or linear correlations of the sample values of the surrogates ($D_{Si}$).

14. Device according to Claim 13, in which the processor is configured in such a way that a significance (S) is calculated from the differences of the nonlinear correlations and/or linear correlations of the sample values of the time series and the nonlinear correlations and/or linear correlations of the sample values of the surrogates ($D_{Si}$), according to the rule

$$S = \frac{|D_0 - \mu_S|}{\sigma_S}$$

in which

$\mu_S$ denotes a mean value of the nonlinear correlations of the sample values of the surrogates ($D_{Si}$),
$\sigma_S$ denotes a variance of the nonlinear correlations of the sample values of the surrogates ($D_{Si}$).

15. Device according to Claim 14, in which the processor is configured in such a way that the mean value ($\mu_S$) of the nonlinear correlations and/or linear correlations of the sample values o.f the surrogates ($D_{Si}$) is determined according to the rule

$$\mu_S = \frac{1}{N} \sum_{i=1}^{N} D_{Si} .$$

16. Device according to Claim 14 or 15, in which the processor is configured in such a way that the variance of the nonlinear correlations and/or linear correlations of the sample values of the surrogates ($D_{Si}$) is determined according to the rule

$$\sigma_S = \sqrt{\frac{1}{N} \sum_{i=1}^{N} (D_{Si} - \mu_S)^2} .$$

17. Device according to one of Claims 13 to 16, in which the processor is configured in such a way that the classification is carried out by [lacuna] the time series in a first time series type, by which an existing stochastic structure between the sample values of the time series is described, or a second time series type, by which non-existent stochastic structures between the sample values of the time series are described.

18. Device according to one of Claims 14 to 17, in which the processor is configured in such a way that the time series is classified as the first time series type if the significance (S) has a value which is greater than a predeterminable limit.

19. Device according to one of Claims 13 to 18, in which the processor is configured in such a way that moments of at least second order are taken into account in the cumulant-based method for calculating the nonlinear correlations.

20. Device according to one of Claims 13 to 19, in which only a predeterminable number of sample values of the time series, which lie chronologically before a reference time, are taken into account.

21. Device according to one of Claims 13 to 20, in which the time series is a measured electrocardiogram (ECG).

22. Device according to one of Claims 13 to 20, in which the time series is a measured electroencephalogram (EEG).

**23.** Device according to one of Claims 13 to 20, in which the time series is a measured signal by which an oxygen pressure profile of a brain is described.

**24.** Use of the device according to one of Claims 13 to 23 to select training data for a neural network from the sample values of the time series, in which only the sample values which have nonlinear correlations and/or linear correlations are used as training data.

**Revendications**

**1.** Procédé pour la classification d'une série chronologique d'un signal électrique par un ordinateur, laquelle série comporte un nombre prédéfinissable de valeurs de balayage,

- dans lequel on détermine un nombre prédéfinissable de substituts ($S_i$, i=1... N) pour la série chronologique (102),
- dans lequel on détermine pour la série chronologique et pour les substituts ($S_i$), des corrélations non linéaires et/ou des corrélations linéaires (dépendance statistique) entre les valeurs de balayage de la série chronologique ($D_0$), et des corrélations non linéaires entre les valeurs de balayage des substituts ($D_{Si}$), à l'aide d'une méthode basée sur la cumulation (103),
- dans lequel on classe la série chronologique selon les différences entre les corrélations non linéaires et/ou les corrélations linéaires des valeurs de balayage de la série chronologique ($D_0$) et les corrélations non linéaires et/ou les corrélations linéaires des valeurs de balayage des substituts ($D_{Si}$) (104).

**2.** Procédé selon la revendication 1, dans lequel on détermine une signification (S) à partir des différences entre les corrélations non linéaires et/ou les corrélations linéaires des valeurs de balayage de la série chronologique et les corrélations non linéaires et/ou les corrélations linéaires des valeurs de balayage des substituts ($D_{Si}$), selon la règle :

$$S = \frac{|D_0 - \mu_S|}{\sigma_S}$$

$\mu_S$ étant une moyenne des corrélations non linéaires des valeurs de balayage des substituts ($D_{Si}$),
$\sigma_S$ étant une variance des corrélations non linéaires des valeurs de balayage des substituts ($D_{Si}$).

**3.** Procédé selon la revendication 2, dans lequel on détermine la moyenne ($\mu_S$) des corrélations non linéaires et/ou des corrélations linéaires des valeurs de balayage des substituts ($D_{Si}$) selon la règle :

$$\mu_S = \frac{1}{N}\sum_{i=1}^{N} D_{Si}.$$

**4.** Procédé selon la revendication 2 ou 3, dans lequel on détermine la variance des corrélations non linéaires et/ou des corrélations linéaires des valeurs de balayage des substituts ($D_{Si}$) selon la règle:

$$\sigma_S = \sqrt{\frac{1}{N}\sum_{i=1}^{N}\left(D_{Si} - \mu_S\right)^2}.$$

**5.** Procédé selon l'une des revendications 1 à 4, dans lequel on effectue la classification en classant la série chronologique dans un premier type de série chronologique servant à décrire une structure stochastique existante entre les valeurs de balayage de la série chronologique, ou dans un deuxième type de série chronologique servant à décrire des structures stochastiques non existantes entre les valeurs de balayage de la série chronologique.

**6.** Procédé selon l'une des revendications 2 à 5, dans lequel on classe la série chronologique comme le premier type de série chronologique si la signification (S) présente une valeur supérieure à une limite prédéfinissable.

7. Procédé selon l'une des revendications 1 à 6, dans lequel des cumulateurs de moments au moins de deuxième ordre sont pris en compte dans la méthode basée sur la cumulation pour la détermination des corrélations non linéaires.

8. Procédé selon l'une des revendications 1 à 7, dans lequel seul un nombre prédéfinissable de valeurs de balayage antérieures à un moment de référence est pris en compte par la série chronologique.

9. Procédé selon l'une des revendications 1 à 8, dans lequel la série chronologique est un signal mesuré d'un électrocardiogramme (E.C.G.).

10. Procédé selon l'une des revendications 1 à 8, dans lequel la série chronologique est un signal mesuré d'un électroencéphalogramme (E.E.G.)

11. Procédé selon l'une des revendications 1 à 8, dans lequel la série chronologique est un signal mesuré décrivant l'évolution d'une pression d'oxygène d'un cerveau.

12. Utilisation du procédé selon l'une des revendications 1 à 11 pour sélectionner parmi les valeurs de balayage de la série chronologique des données d'entraînement pour un réseau neuronal, consistant à utiliser comme données d'entraînement seules les valeurs de balayage ayant des corrélations non linéaires et/ou des corrélations linéaires.

13. Appareil pour la classification d'une série chronologique d'un signal électrique, laquelle série comporte un nombre prédéfinissable de valeurs de balayage, à l'aide d'un processeur configuré de façon à pouvoir effectuer les étapes suivantes :

- on détermine un nombre prédéfinissable de substituts ($S_i$, i=1... N) pour la série chronologique,
- on détermine pour la série chronologique et pour les substituts ($S_i$), des corrélations non linéaires et/ou des corrélations linéaires (dépendance statistique) entre les valeurs de balayage de la série chronologique ($D_0$), et des corrélations non linéaires entre les valeurs de balayage des substituts ($D_{Si}$), à l'aide d'une méthode basée sur la cumulation,
- on classe la série chronologique selon les différences entre les corrélations non linéaires et/ou les corrélations linéaires des valeurs de balayage de la série chronologique ($D_0$) et les corrélations non linéaires et/ou les corrélations linéaires des valeurs de balayage des substituts ($D_{Si}$).

14. Appareil selon la revendication 13, dans lequel le processeur est configuré de façon telle que l'on détermine une signification (S) à partir des différences entre les corrélations non linéaires et/ou les corrélations linéaires des valeurs de balayage de la série chronologique et les corrélations non linéaires et/ou les corrélations linéaires des valeurs de balayage des substituts ($D_{Si}$), selon la règle :

$$S = \frac{|D_0 - \mu_S|}{\sigma_S}$$

$\mu_S$ étant une moyenne des corrélations non linéaires des valeurs de balayage des substituts ($D_{Si}$),
$\sigma_S$ étant une variance des corrélations non linéaires des valeurs de balayage des substituts ($D_{Si}$).

15. Appareil selon la revendication 14, dans lequel le processeur est configuré de façon telle que l'on détermine la moyenne ($\mu_S$) des corrélations non linéaires et/ou des corrélations linéaires des valeurs de balayage des substituts ($D_{Si}$) selon la règle :

$$\mu_S = \frac{1}{N} \sum_{i=1}^{N} D_{Si}.$$

16. Appareil selon la revendication 14 ou 15, dans lequel le processeur est configuré de façon telle que l'on détermine la variance des corrélations non linéaires et/ou des corrélations linéaires des valeurs de balayage des substituts

($D_{Si}$) selon la règle :

$$\sigma_S = \sqrt{\frac{1}{N}\sum_{i=1}^{N}\left(D_{Si} - \mu_S\right)^2}\,.$$

17. Appareil selon l'une des revendications 13 à 16, dans lequel le processeur est configuré de façon telle que l'on effectue la classification en classant la série chronologique dans un premier type de série chronologique servant à décrire une structure stochastique existante entre les valeurs de balayage de la série chronologique, ou dans un deuxième type de série chronologique servant à décrire des structures stochastiques non existantes entre les valeurs de balayage de la série chronologique.

18. Appareil selon l'une des revendications 14 à 17, dans lequel le processeur est configuré de façon telle que l'on classe la série chronologique comme le premier type de série chronologique si la signification (S) présente une valeur supérieure à une limite prédéfinie.

19. Appareil selon l'une des revendications 13 à 18, dans lequel le processeur est configuré de façon telle que des cumulateurs de moments au moins de deuxième ordre sont pris en compte dans la méthode basée sur la cumulation pour la détermination des corrélations non linéaires.

20. Appareil selon l'une des revendications 13 à 19, dans lequel le processeur est configuré de façon telle que seul un nombre prédéfinissable de valeurs de balayage antérieures à un moment de référence est pris en compte par la série chronologique.

21. Appareil selon l'une des revendications 13 à 20, dans lequel la série chronologique est un signal mesuré d'un électrocardiogramme (E.C.G.).

22. Appareil selon l'une des revendications 13 a 20, dans lequel la série chronologique est un signal mesuré d'un électroencéphalogramme (E.E.G.)

23. Appareil selon l'une des revendications 13 à 20, dans lequel la série chronologique est un signal mesuré décrivant l'évolution d'une pression d'oxygène d'un cerveau.

24. Utilisation de l'appareil selon l'une des revendications 13 à 23 pour sélectionner parmi les valeurs de balayage de la série chronologique des données d'entraînement pour un réseau neuronal, consistant à utiliser comme données d'entraînement seules les valeurs de balayage ayant des corrélations non linéaires et/ou des corrélations linéaires.

# FIG 1

101 — Messung einer Zeitreihe mit einer vorgebbaren Anzahl von Abtastwerten

102 — Bestimmung einer vorgebbaren Anzahl von Surrogaten der Zeitreihe

103 — Bestimmung nichtlinearer Korrelationen mit einer kumulanzbasierten Methode für die Abtastwerte der Zeitreihe und die Abtastwerte der Surrogate

104 — Klassifikation der Zeitreihe anhand der Unterschiede der nichtlinearen Korrelationen der Abtastwerte der Zeitreihe und der Abtastwerte der Surrogate

# FIG 2

101 — Messung einer Zeitreihe mit einer vorgebbaren Anzahl von Abtastwerten

102 — Bestimmung einer vorgebbaren Anzahl von Surrogaten der Zeitreihe

103 — Bestimmung nichtlinearer Korrelationen mit einer kumulanzbasierten Methode für die Abtastwerte der Zeitreihe und die Abtastwerte der Surrogate

201 — Bestimmung einer Signifikanz

202 — Signifikanz > Schwelle ?

Nein

Ja

204 — Zeitreihe wird als ein erster Zeitreihentyp klassifiziert

203 — Zeitreihe wird als ein zweiter Zeitreihentyp klassifiziert

# FIG 3

```
                              ┌─────────── 301
                    ┌──────────────────┐
                    │    Arten von     │
                    │    Zeitreihen    │
                    └──────────────────┘
           ┌──────────┬──────────┴──────────┬──────────┐
           ▼          ▼                      ▼          ▼
    ┌──────────┐ ┌──────────┐ ┌──────────┐ ┌──────────┐
    │          │ │          │ │ Verlauf der│ │ Verlauf der│
    │          │ │          │ │   lokalen  │ │   lokalen  │
    │   EKG-   │ │   EEG-   │ │Sauerstoff- │ │Sauerstoff- │ ......
    │  Signal  │ │  Signal  │ │ spannung   │ │ spannung   │
    │          │ │          │ │   eines    │ │   eines    │
    │          │ │          │ │  Gehirns   │ │  Gehirns   │
    └──────────┘ └──────────┘ └──────────┘ └──────────┘
           302        303          304          305
```

# FIG 4